# EUROPÄISCHE PATENTSCHRIFT

(11) **EP 0 546 144 B1**
(45) Veröffentlichungstag und Bekanntmachung des Hinweises auf die Patenterteilung: **13.03.1996**
(21) Anmeldenummer: 92913732.1
(22) Anmeldetag: 01.07.1992
(51) Int. Cl.: C08J 9/26, C12N 5/00

(54) **THERMISCH STERILISIERBARER PORÖSER TRÄGERKÖRPER FÜR BIOKATALYSATOREN**
HEAT-STERILISABLE POROUS SUBSTRATE FOR BIO-CATALYSTS
SUBSTRAT POREUX THERMOSTERILISABLE POUR BIOCATALYSEURS

(30) Priorität: 02.07.1991 AT 1322/91
(43) Veröffentlichungstag der Anmeldung: 16.06.1993
(73) Patentinhaber: Pharmacia Biotech AB, S-751 82 Uppsala (SE)
(72) Erfinder: KATINGER, Hermann, W., D., A-1190 Wien (AT); RAUSCHERT, Bartold, D-8641 Steinbach am Wald (DE); BLÜML, Gerald, A-1160 Wien (AT); ZACH, Nicolaus, A-1080 Wien (AT); REITER, Manfred, A-1160 Wien (AT); GAIDA, Theodor, A-1020 Wien (AT)
(74) Vertreter: Nöth, Heinz, Dipl.-Phys.
(86) Internationale Anmeldenummer: AT9200081
(87) Internationale Veröffentlichungsnummer: WO9301231

(56) Entgegenhaltungen:
- EP-A- 0 158 925
- GB-A- 2 109 389

## Beschreibung

Die Erfindung betrifft einen partikelförmigen Träger zum Kultivieren von Zellen gemäß dem Oberbegriff des Patentanspruchs 1.

Derartige Träger dienen insbesondere zur Kultivierung von Mikroorganismen in Bioreaktorsystemen, und zwar sowohl in gerührten, gepackten und fluidisierten Systemen.

Es sind bereits einige Trägertypen für die in vitro-Kultur von Mikroorganismen bekannt, welche sowohl für bakterielle als auch für tierische Zellkulturen verwendet werden. Für die Technologie der Kultivierung von Säugetierzellen bedient man sich in der Regel des unter dem Namen "Cytodex"^{R} erhältlichen Carriers (welcher von der Fa. Pharmacia in den Handel gebracht wird). Diese Carrier sind zwar sphärischen Typs, sind jedoch nicht porös und haben daher den Nachteil, daß sie nur für strikt adhärente Zelltypen eingesetzt werden. Für in Suspension wachsende, also nicht adhärente Zellen, ist dieser Typ von Carriern nicht geeignet, da z.B. bei Fließbettsystemen ein Auswaschen der Zellen erfolgen kann. Außerdem liegen bei den genannten Carriern die erreichbaren Zelldichten bei max. 10⁻⁷ Zellen/ml.

Weiters ist bereits ein poröser Carrier auf der Basis von Gelatine bekannt, welcher sich für bewegte Substrate aufweisende Bioreaktoren eignet. Ebenso ist z.B. aus dem Dokument US-A-4, 863, 856 ein aus Collagen hergestellter, mit inertem Füllstoff beschwerter Carrier bekannt, bei welchem bevorzugt Collagen vom Typ I bovinen Ursprungs verwendet wird. Sowohl den Gelatine- als auch den Collagen-Carriertypen ist gemeinsam, daß ihr Ausgangsmaterial in der Regel tierischer Natur ist. Dies bringt mit sich, daß diese Ausgangsmaterialien heterogen beschaffen sind, d.h. daß eine gleichbleibende Qualität und gleichbleibende Eigenschaften nur selten gegeben sind. Es ist also ein sehr hoher Aufwand im Hinblick auf die Qualitätskontrolle erforderlich. Außerdem ist eine thermische Sterilisierbarkeit bei diesen Materialien nur bedingt gegeben.

Es sind auch bereits thermisch sterilisierbare poröse Trägerkörper der eingangs genannten An bekannt, bei welchen als Trägerkörper ein poröses Sinterglas eingesetzt wird. Es handelt sich dabei um die unter dem Markennamen Siran^{R} der Fa. Schott in den Handel gebrachten Sinterglaskörper. Diese bekannten Sinterglaskörper sind als Füllkörper konzipiert und sind daher nur in Festbettreaktoren einsetzbar, da dort ein ruhendes Bett, also keine Relativbewegung zwischen den einzelnen Trägerkörpern vorkommt. Bei Fließbettreaktoren bzw. auch bei konventionell gerührten Reaktoren sind jedoch die Glassinterkörper nur begrenzt einsetzbar, da aufgrund der gegenseitigen Relativbewegung der Körner ein Abrieb entsteht, der für die zu kultivierenden tierischen Zellen schädlich ist, und außerdem aufgrund der mechanischen Einwirkungen auf die Oberflächen des Sinterglaskörpers diese die gewünschte Struktur verlieren können.

Das Dokument WO 86/05811 beschreibt einen Mikroschwamm zum Immobilisieren von bioaktiven Materialien wie Enzymen, Mikroorganismen, Zellen usw., mit einer porösen biostabilen Matrix aus einem biokompatiblen Polymer, das einen inerten beschwerenden Stoff wie Metall oder Legierungen oder Oxide davon oder Keramik enthält und eine durchschnittliche Partikelgröße von 100 bis 1000 µm und ein spezifisches Gewicht von mehr als 1,05 aufweist. Die Poren sind nach außen offen und haben ein Porenvolumen von 70 bis 98 % und eine durchschnittliche Porengröße von 1 bis 150 µm. Das Polymer besteht vorzugsweise aus hochvernetztem lyophilisierten Collagen.

Das Dokument EP-A-0 222 718 beschreibt makroporöse Partikel, die als Träger zum Kultivieren von Zellen verwendbar sind und durch Vermischen einer festen, flüssigen oder gasförmigen, Hohlräume erzeugenden Verbindung mit einer wässrigen Lösung einer matrixbildenden Verbindung, Dispergieren der Mischung in Wasser zum Bilden von Partikeln, Unlöslichmachen der Matrix durch Abkühlen, Vernetzen oder Polymerisieren und Entfernen der die Hohlräume erzeugenden Verbindung hergestellt werden. Die matrixbildende Verbindung wird ausgewählt aus Proteinen, Polysacchariden und synthetischen Polymeren wie Polyacrylamid. Die Hohlräume erzeugende Verbindung kann z.B. aus Calciumcarbonat bestehen, das nach dem Unlöslichmachen der Matrix mit Säure entfernt wird. Die Hohlräume verleihen den Partikeln eine größere spezifische Oberfläche und es kann das Innere der Partikel für das Kultivieren von Zellen verwendet werden, die einer Verankerung bedürfen. Die Partikel weisen eine Größe von 10 bis 500 µm und die Hohlräume einen Durchmesser von 1 bis 50 µm auf.

Das Dokument EP-A-0 274 587 beschreibt die Durchführung biotechnischer Verfahren in Fest- oder Fließbetten unter Verwendung von sphärischen Sinterkörpern aus offenporigem anorganischen Material wie Sinterglas, dessen durchgehende Poren mehrmals größer als die eingesetzten Mikroorganismen oder tierischen Zellen sind und einen Flüssigkeits- oder Gasaustausch mit dem Inneren des Trägermaterials zulassen, wobei ein Wachstum der Mikroorganismen innerhalb der Poren stattfindet. Die Dichten und Durchmesser der sphärischen Partikel sind derart gewählt, daß in einem Fließbettreaktor die aus den Partikeln herauswachsenden Zellen abgeschert werden und deren Wachstum auf das Innere der Partikel beschränkt wird.

Gemäß dem Dokument GB-A-2 109 389 sind Füllstoff enthaltende Polymerpartikel, die u.a. auch als Träger für biologische Substanzen Verwendung finden, aufgrund des Vorhandenseins des Füllstoffes an der Oberfläche nicht geeignet als Träger zum Adsorbieren von oder Kombinieren mit biologischen Substanzen wie immunoreaktiven Substanzen, Enzymen oder Zellen. Es werden zu diesem Zweck Füllstoff enthaltende Polymerpartikel vorgesehen, deren Oberfläche mit einem Polymer ohne Füllstoff überzogen ist. Die gefüllten Partikel werden durch eine Suspensionspolymerisation einer ethylenisch ungesättigten Verbindung in Gegenwart des Füllstoffes hergestellt, wonach der Überzug durch eine Radikalpolymerisation einer ethylenisch ungesättigten Verbindung in wässriger Phase in Gegenwart der dispergierten gefüllten Partikel hergestellt wird. Die gefüllten und ungefüllten Partikel weisen Durchmesser von 0,1 bis 2000 µm bzw. 0,1 bis 3000 µm auf.

Der Erfindung liegt die Aufgabe zugrunde, einen partikelförmigen Träger gemäß dem Oberbegriff des Patentanspruchs 1 zu schaffen, der mit steuerbaren Eigenschaften und gleichbleibender Qualität herstellbar, bei jeder Art von Bioreaktoren einsetzbar und thermisch sterilisierbar ist.

Die erfindungsgemäße Lösung dieser Aufgabe ergibt sich aus dem kennzeichnenden Teil des Patentanspruchs 1.

Der für Biokatalysatoren geeignete poröse Trägerkörper des erfindungsgemäßen Trägers ist thermisch sterilisierbar und mit großem offenen Porenvolumen versehen.

Der Trägerkörper besitzt als Trägermaterial mit Füllstoff versehenes, vernetztes Polyolefin, das nicht tierischen Ursprungs ist, wobei die Dichte des Trägerkörpers größer als 1 ist. Der Trägerkörper kann aufgrund seines Aufbaues mit genau gesteuerten Eigenschaften und gleichbleibender Qualität hergestellt werden, wobei aufgrund der Dichte dieser Trägerkörper auch in Fließbettreaktoren bevorzugt einsetzbar ist.

Das Polyolefin ist vorzugsweise ein HDPE, ein Polypropylen od.dgl. Diese Materialien haben neben der leichten Steuerung der Eigenschaften auch den Vorteil, daß sie entsprechend thermisch sterilisierbar sind. Dabei kann der anorganische Füllstoff vorzugsweise Kreide sein. Durch den anorganischen Füllstoff können die Oberflächeneigenschaften und auch die spezifische Dichte entsprechend gesteuert werden, wobei insbesondere ein calciumhaltiger Füllstoff, also Kreide, den Vorteil hat, daß durch die Calciumionen an der Oberfläche der Trägerkörper bzw. in den Poren ein besonders gutes Verhalten der Trägerkörper gegenüber den Mikroorganismen erreicht wird. Dabei kann das Verhältnis von Kreide zu Polyethylen von etwa 1 : 1,5 bis 1 : 4,5, vorzugsweise etwa 1 : 3, betragen. Auch das Extrudieren der Polyolefine wird durch diese Trägerstoffe insbesondere in der angegebenen Konzentration erleichtert. Zur Steuerung der Dichte der Trägerkörper kann zusätzlich ein Schwermetallsalz eingesetzt werden, wobei auf biologisch unbedenkliche Weise die Dichte der Trägerkörper steuerbar ist.

Zur Herstellung des erfindungsgemäßen Trägers wird das Polymermaterial mit den anorganischen Komponenten gemischt und anschließend. extrudiert. Danach werden die leicht löslichen anorganischen Bestandteile extrahiert und es bleibt eine offenporige Polymermatrix erhalten, wobei je nach Größe der verwendeten anorganischen Komponente die Größe der Poren des Carriers variiert werden kann. Die offenen Poren liegen im Bereich von 1 - 300 µm, das offene Porenvolumen liegt zwischen 60 und 85 % und die Partikelgröße zwischen 0,1 und 2 mm. Der Anteil an löslicher anorganischer Substanz liegt dabei bei.etwa 50-70 Volumsprozent, und die Körnung derselben bei 1 - 300 µm. Als leicht lösliche anorganische Bestandteile können vorzugsweise Cl- und SO₄-Salze eingesetzt werden, z.B. die Alkali- und Erdalkalisalze davon, also z.B. Kaliumchlorid und Natriumchlorid. Statt der löslichen anorganischen Bestandteile können auch Treibmittel eingesetzt werden, jedoch muß bei den Treibmitteln sichergestellt werden, daß die Trägerkörper nach außen offene Poren aufweisen.

Als nicht lösliche Füllkörper werden zur Erhöhung der spezifischen Dichte des Polymers anorganische Fullstoffe eingesetzt, wobei sich insbesondere im Hinblick auf die Extrusion Kreide besonders anbietet. Außerdem werden durch das Vorhandensein von Calciumionen auf der Polymeroberfläche Wachstumsbedingungen geschaffen, die 5 insbesondere das Wachstum tierischer Zellen an der Oberfläche der Trägerkörpers begünstigen. Die Bildung extrazellulärer Matrix wird durch das Calcium gefördert (siehe Ruoslahti und Pierschbacher, 1987; Pytela et al 1987; Chakravarti et al 1990).

Die erfindungsgemäßen Trägerkörper weisen auch an der Oberfläche Calciumionen auf, welche vor allem durch die Beimischung von Kreide vorhanden sind.

Die Calciumionen dienen der Förderung des Mikroorganismenwachstums. Die Porenstruktur der Trägerkörper ist zu 50 - 60 % offen, wodurch eine sehr hohe spezifische wirksame Oberfläche vorhanden ist. Außerdem ist aufgrund der Ladung der Ionen auch eine entsprechende Ionenaustauschkapazität gegeben, die dem Mikroorganismenwachstum und dem Stoffaustausch mit dem Nährsubstrat zuträglich sind. Die Sinkgeschwindigkeit der Trägerkörper beträgt etwa 150 - 170 cm/min für Fließbettreaktoren. Für Rührkesselfermentoren wird die Dichte der Trägerkörper etwas niedriger eingestellt werden, sodaß die Trägerkörper leichter sind und daher in Rührkesselfermentoren leichter in Schwebe gehalten werden können. Bei Festbettreaktoren hingegen wird eine höhere Sinkgeschwindigkeit, also eine höhere Dichte, angestrebt werden. Die erfindungsgemäßen Trägerkörper sind also für den Einsatz in jeglicher An von Reaktoren vorteilhaft, u.zw. sowohl in gerührten Bioreaktoren als auch in Fließbettreaktoren und Festbettreaktoren. Die Polymermaterialien sind hinsichtlich ihrer physikalischen Eigenschaften für eine breite Palette von Mikroorganismen, insbesondere für adhärente und nicht adhärente Säugetierzellen, sowie auch für Pflanzenzellen geeignet. Außerdem können aufgrund der Oberflächenladung auch sonstige Biokatalysatoren direkt an der Oberfläche der Trägerkörper immobilisiert werden. Die Zellen werden in der porösen Struktur der Trägerkörper festgehalten (immobilisiert), wodurch die Erzielung von sehr hohen Zelldichten möglich ist. Auch ist eine Autoklavierbarkeit, also Sterilisierbarkeit der Trägerkörper, möglich und zwar bis zu 30 min bei 120°C. Dadurch können die Trägerkörper direkt mit der gesamten Anlage sterilisiert werden.

Bei einem bevorzugten Herstellungsverfahren eines erfindungsgemäßen Trägers wird folgende Rezeptur eingesetzt:

50 % lösbares anorganisches Salz, Korngröße 0,1 - 0,3 mm, 12,5 % Kreide in Pulverform, 37,5 % Polyethylen (HDPE).

Die Dichte des HDPE ist 0.962 g/cm³ und der Schmelzindex desselben nach 190/2 beträgt 6 g/10 min.

Zur Herstellung wird der Kunststoff HDPE fein gemahlen und aus allen drei Komponenten danach eine gründliche Vormischung hergestellt. Diese Mischung wird mittels gleichlaufenden Doppelschneckenextrudern extrudiert, u.zw. bei einem Druck von etwa 300 bar und bei einer Verarbeitungstemperatur von etwa 180°C. Der Extrusionsstrang wird dann mittels eines Kopfgranulators in einem Wassernebel abgeschlagen und die abgeschlagenen Granulatteile werden in einem Wasserbad gekühlt, worauf dann das Salz ausgewaschen wird. Als nächsten Schritt werden dann die Granulatteilchen in einer Zentrifuge getrocknet und, falls erforderlich, für eine Verwendung autoklaviert.

In den beiliegenden Fotos werden die Porenstruktur der Trägerkörper und das Fließverhalten des Bioreaktors gezeigt. Foto 1a zeigt die Porenstruktur des Carriers, 1b die Porenstruktur des Carriers im Detail und Foto 1c das Fließverhalten im Bioreaktor. Foto 2a zeigt adhärente tierische Zellen (CHO), die auf dem porösen Carrier kultiviert sind. Das Foto 2b zeigt Suspensionszellen (Hybridoma-Zellen) auf dem porösen Carrier.

Zusammenfassend kann festgestellt werden, daß die Vorteile des erfindungsgemäßen Trägers in folgenden Eigenschaften liegen:
Definierte Porenstruktur
definierte spezifische Gewichte (ergibt ideales Fließverhalten in gerührten und in Fließbettreaktoren)
mechanische Beständigkeiten
Beständigkeit gegenüber Säuren und Laugen
Beständigkeit gegenüber enzymatischem Abbau, daher Langzeitstabilität
toxische Rückstände können ausgeschlossen werden
Eignung zur Verwendung in gerührten und nichtgerührten Bioreaktoren
Autoklavierbarkeit bei 120°C
Oberflächenmodifizierung ist möglich.

Zitierte Literatur:
Ruoslahti, E. und Pierschbacher, M.D. 1987. Science 238:491.
Pytela, R., Pierschbacher, M.D. Argraves, W.S. Suziki, S.und Ruoslahti, E.
1987. Methods Enzymol. 144:475.

Chakravarti, S., Tam. M.F. und Chung, A.E.1990. The Journal of Biol.Chem. 265:10597.

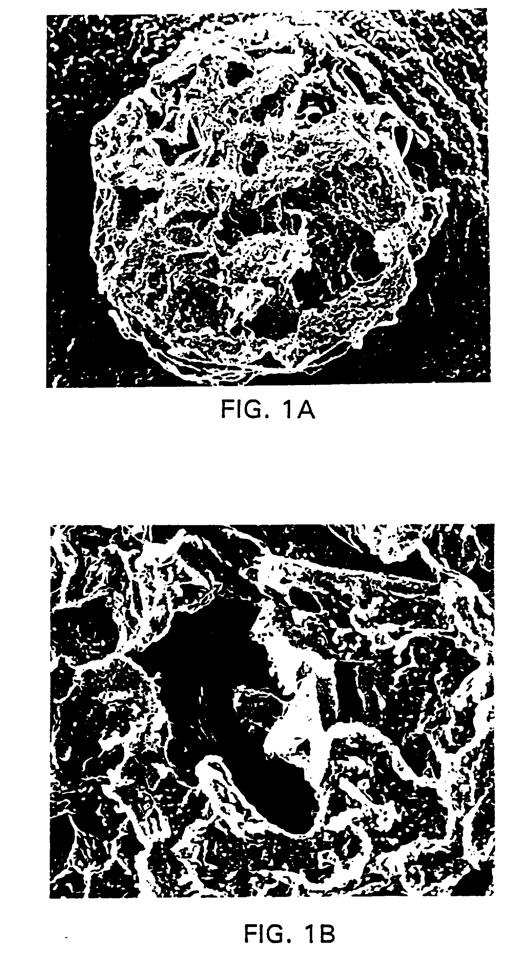

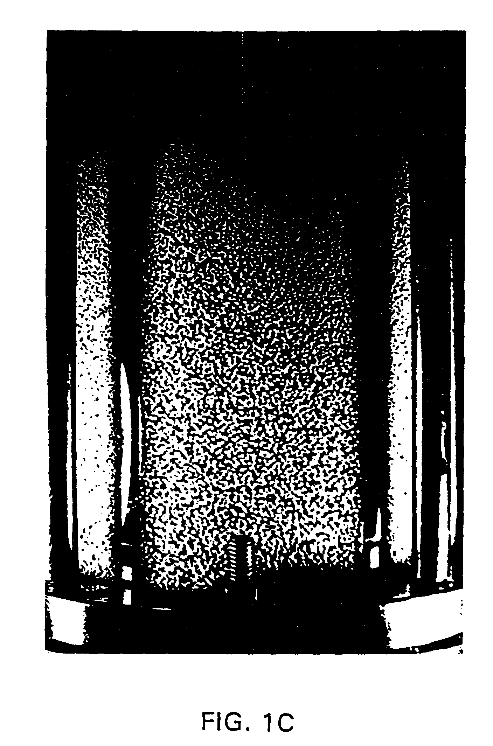

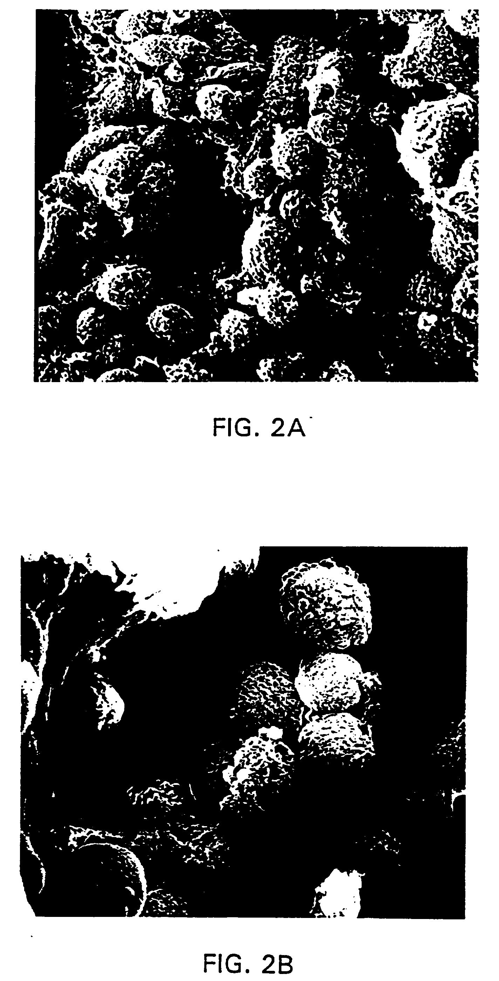

## Patentansprüche

1. Partikelförmiger Träger zum Kultivieren von Zellen, dessen Dichte größer als 1 g/cm³ ist, der als Trägermaterial mit anorganischem Füllstoff versehenes Polymer besitzt und der offene Poren und eine poröse Struktur zum Festhalten von Zellen aufweist, **dadurch gekennzeichnet,** daß das Polymer ein Polyolefin ist.

2. Träger nach Anspruch 1, **dadurch gekennzeichnet,** daß dessen Partikelgröße zwischen 0,1 und 2 mm liegt, das offene Porenvolumen zwischen 60 und 85 % liegt und die Größe der offenen Poren im Bereich von 1 bis 300 µm liegt.

3. Träger nach Anspruch 1 oder 2, **dadurch gekennzeichnet,** daß das Polyolefin ein HDPE oder ein Polypropylen ist.

4. Träger nach einem der Ansprüche 1 bis 3, **dadurch gekennzeichnet,** daß der Füllstoff Kreide ist.

5. Träger nach Anspruch 4, **dadurch gekennzeichnet**, daß das Polyolefin Polyethylen ist und das Verhältnis von Kreide zu Polyethylen 1:1,5 bis 1:4,5 beträgt.

6. Träger nach Anspruch 5, **dadurch gekennzeichnet,** daß das Verhältnis von Kreide zu Polyethylen 1:3 beträgt.

7. Träger nach einem der Ansprüche 1 bis 6, **dadurch gekennzeichnet,** daß dieser zusätzlich ein Schwermetallsalz enthält.

8. Verfahren zur Herstellung eines partikelförmigen Trägers einer der Ansprüche 1 bis 7, **dadurch gekennzeichnet,** daß ein Polyolefin mit einem nicht löslichen anorganischen Füllstoff und 50 bis 70 Vol.-% einer leicht löslichen anorganischen Komponente vermischt, extrudiert und granuliert wird, wonach die leicht löslichen anorganischen Bestandteile extrahiert werden.

9. Verwendung eines partikelförmigen Trägers einer der Ansprüche 1 bis 7 zur Züchtung von Mikroorganismen.

## Claims

1. A particulate carrier for cultivating cells, said carrier having a density greater than 1 g/cm³, and possessing as carrier material a cross-linked polymer provided with inorganic filler, and having open pores and a porous structure for retaining cells, **characterized in that** the polymer is a polyolefine.

2. A carrier according to claim 1, **characterized in that** the particle size thereof is between 0.1 and 2 mm, the open pore volume is between 60 and 85 %, and the size of the open pores is in the range from 1 to 300 µm.

3. A carrier according to claim 1 or 2, **characterized in that** the polyolefine is a HDPE or a polypropylene.

4. A carrier according to any one of claims 1 to 3, **characterized in that** the filler is chalk.

5. A carrier according to claim 4, **characterized in that** the polyolefine is polyethylene, and the ratio of chalk to polyethylene amounts to 1:1.5 to 1:4.5.

6. A carrier according to claim 5, **characterized in that** the ratio of chalk to polyethylene amounts to 1:3.

7. A carrier according to any one of claims 1 to 6, **characterized in that** it additionally contains a heavy metal salt.

8. A method for producing a particulate carrier of any one of the claims 1 to 7, **characterized in that** a polyolefine is mixed with a non-soluble inorganic filler and 50 to 70 % by volume of an easily soluble inorganic component, extruded and granulated, after which the easily soluble inorganic constituents are extracted.

9. Use of a particulate carrier of any one of the claims 1 to 7 for cultivating microorganisms.

## Revendications

1. Substrat sous forme de particules pour la culture de cellules, dont la masse volumique est supérieure à 1 g/cm³, qui possède comme matière substrat un polymère pourvu de matière de charge minérale et qui présente des pores ouverts et une structure poreuse pour retenir les cellules, caractérisé en ce que le polymère est une polyoléfine.

2. Substrat selon la revendication 1, caractérisé en ce que sa grosseur de particules est comprise entre 0,1 et 2,0 mm, la porosité ouverte est comprise entre 60 et 85 % et la grosseur des pores ouverts est comprise entre 1 et 300 µm.

3. Substrat selon l'une des revendications 1 à 2, caractérisé en ce que la polyoléfine est un polyéthylène haute densité ou un polypropylène.

4. Substrat selon l'une des revendications 1 à 3, caractérisé en ce que la matière de charge est de la craie.

5. Substrat selon la revendication 4, caractérisé en ce que la polyoléfine est un polyéthylène et le rapport de la craie au polyéthylène est de 1/1,5 à 1/4,5.

6. Substrat selon la revendication 5, caractérisé en ce que le rapport de la craie au polyéthylène est de 1/3.

7. Substrat selon l'une des revendications 1 à 6, caractérisé en ce qu'il contient en plus un sel de métal lourd.

8. Procédé de fabrication d'un substrat sous forme de particules selon l'une des revendications 1 à 7, caractérisé en ce qu'on mélange une polyoléfine avec une matière de charge minérale insoluble et 50 à 70 % en volume d'un constituant minéral facilement soluble, extrude et granule le mélange, puis extrait les constituants minéraux facilement solubles.

9. Utilisation d'un substrat sous forme de particules selon l'une des revendications 1 à 7 pour la culture de micro-organismes.
